# EUROPEAN PATENT SPECIFICATION

(11) **EP 1 886 698 B1**
(45) Date of publication and mention of the grant of the patent: **16.02.2011**
(21) Application number: 06016652.7
(22) Date of filing: 09.08.2006
(51) Int. Cl.: A61L 15/46, A61F 13/15

(54) **Absorbent articles including an improved odour control system**
Absorbierende Artikel mit einem verbesserten Geruchskontrollesystem
Articles absorbants dotés d' un système amélioré de suppression des mauvaises odeurs

(43) Date of publication of application: 13.02.2008
(73) Proprietor: The Procter & Gamble Company, Cincinnati, OH 45202 (US)
(72) Inventor: Sierri, Giancarlo, 64030 Montefino (Teramo) (IT); Bellucci, Remo, 65010 Spoitore (Pescara) (IT); Caputi, Mariangela, 70056 Molfetta (Bari) (IT); D'Ercole, Luigia, 65015 Montesilvano (Pescara) (IT)
(74) Representative: Briatore, Andrea

(56) References cited:
- EP-A- 0 510 619
- EP-A- 1 275 404
- EP-A1- 1 250 914
- WO-A-97/46189
- WO-A-98/51248
- WO-A2-98/26808
- WO-A2-2006/038931
- US-A1- 2003 135 172

## Description

### FIELD OF THE INVENTION

The present invention relates to absorbent articles which are provided with an odour control system The odour control system comprises classes of odour control materials, wherein one class comprises materials with low volatility and a second class comprises materials with high volatility. The classes of odour control materials may be selected to provide a synergistic effect in terms of malodour reduction, and the odour control system is positioned in the absorbent article in order to provide an improved odour control action over a longer time, starting from the moment the article is taken from its package and prepared for use.

### BACKGROUND OF THE INVENTION

Absorbent articles of personal hygiene are known in the art Typical examples include sanitary napkins, panty liners, adult incontinence articles, infant diapers, paper towels, bath tissue and facial tissue. Such articles are often used to absorb and retain bodily fluids and other exudates excreted by the human body Particularly, articles for personal feminine hygiene such as sanitary napkins, panty liners and articles for light adult incontinence are used by applying them to the crotch portion of an undergarment by means of adhesive means, usually an adhesive layer provided onto the backsheet garment side, and protected by a release layer which is removed before use in order to expose the adhesive Typically, such fluids and exudates are perceived as malodorous and offensive Therefore, methods and materials for controlling and reducing malodours in absorbent articles have been developed Some examples are discussed hereinafter

An early, basic reference in this respect is EP 510619 This document discloses a wide variety of materials, which have proven To be effective in certain circumstances in reducing malodours in absorbent articles of personal hygiene. EP 959846 discloses such materials comprising polyacrylate superabsorbers and silica. EP 811387 discloses absorbent articles being provided with a zeolite and silica odour control system EP 963186 discloses an odour control systems comprising zeolites, silica and polyacrylic superabsorbers EP 912149 discloses chelating agents for use in odour control in absorbent articles, particularly polyfunctionally substituted aromatic chelants. All of the above solutions can provide consumer-noticeable degree of malodour reduction in absorbent articles. However, due to the nature of action and the materials chosen only a limited variety of malodorous compounds can be counteracted.

Therefore it is desirable to provide absorbent articles having an odour control system, which acts against a wide variety of malodours in a holistic manner Particularly, it is desirable to provide an absorbent article with an odour control system which is capable of ensuring a sustained action over Time, i.e. starting from the very moment the article is taken from its package, and continuing during its use Desirably the odour control system comprises at least one class of odour control materials having low volatility, and at least another class of odour control materials having high volatility, the odour control system is positioned into the absorbent article such that the high volatility odour control materials are substantially prevented from emanating from the absorbent article until the article is taken from its package Particularly, in articles intended to be applied to the crotch portion of an undergarment by means of an adhesive layer, until the release layer is removed from the article, typically by detaching it from the panty fastening adhesive layer provided onto the absorbent article backsheet.

It is also desirable to provide an absorbent article having an odour control system where the at least one class of materials comprising low volatility odour control materials reduces malodours by acting on malodours or a malodourous substance in the article and the at least other class of materials comprising high volatility odour control materials acts on certain nose receptors, typically to help reduce the perception of malodour, or to provide a pleasant odour.

### SUMMARY OF THE INVENTION

The present invention addresses the above need by providing an absorbent article comprising a liquid permeable topsheet, a backsheet, and an absorbent core comprised between the topsheet and the backsheet, each of the topsheet, backsheet and absorbent core having a body facing side and a garment facing side; the garment facing side of the core also has a core surface area The absorbent article comprises an odour control system comprising first and second classes of odour control materials, wherein the first class comprises at least a material having a Kovat Index (KI) higher than 1500, preferably between 1550 and 1900, and the second class comprises at least a material having a Kovat Index of 1500 or less, preferably between 900 and 1500, more preferably between 1000 and 1400. At least the second class of odour control materials of the odour control system is provided onto the garment facing side of the absorbent core, adjacent to the body facing side of the backsheet, on a surface area of at least 900 mm², preferably of 900 mm² to 2000 mm², more preferably of 900 mm² to 1300 mm², or on a surface area such that the ratio of this surface area and the core surface area is at least 0.08, preferably between 0.08 and 0 2, more preferably between 0.08 and 0.1.

### DETAILED DESCRIPTION OF THE INVENTION

The term "absorbent article" is used herein in a very broad sense including any article able to receive and/or absorb and/or contain and/or retain fluids and/or exudates, especially bodily fluids/bodily exudates. Exemplary absorbent articles in the context of the present invention are disposable absorbent articles

The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). Typical disposable absorbent articles according to The present invention can be absorbent articles for feminine hygiene such as sanitary napkins, panty liners, light incontinence products, or the like Absorbent articles according to the present invention can also encompass diapers, surgical and wound dressings and perspiration pads, incontinence pads

The term "use", as used herein, refers to the period of time that starts when the absorbent article is actually put in contact with the anatomy of a wearer.

By "body fluid" it is meant herein any fluid produced by human body including, but not limited to, perspiration, urine, menstrual fluids, vaginal secretions and the like

The present invention relates to an absorbent article comprising an odour control system, which reduces the malodour generated by a broad range of odorous materials typically occurring in or resulting from the degradation of body fluids and/or materials making up the absorbent article The odour control system herein includes at least two classes of odour control materials, a first class comprises at least an odour control material having a low volatility, and a second class comprises at least an odour control material having a high volatility. In an embodiment of the present invention, one class of odour control materials, typically the first class above, counteracts the malodours of the exudates or other malodorous material (also referred to herein as acting "internally"), whereas typically the second class counteracts such malodours by affecting the receptors in the nose

### Absorbent article

The absorbent article of the present invention can be any kind of absorbent article for personal hygiene known in the art, as described above, particularly an absorbent article for feminine hygiene, and typically comprises a liquid permeable topsheet, a backsheet, and an absorbent core therebetween. Each of these elements, as well as any other optional layer present in the absorbent article, has a body facing side or wearer facing side, and a garment facing side or outer facing side, which correspond to the side facing respectively the body and the garment of the wearer during use of the product.

As it is known in the art, topsheets may be manufactured from a wide range of materials which include, but are not limited to, woven and nonwoven materials; polymeric materials such as apertured formed thermoplastic films, apenured plastic films, and hydroformed thermoplastic films; porous foams; reticulated foams; reticulated thermoplastic films, and thermoplastic scrims. A topsheet is typically a specific separate element in the absorbent article of the present invention, comprising one or more layers; however, in an absorbent article according to the present invention the topsheet is meant to correspond to the layer or element which in use goes in direct contact with the user's body, for example, the topsheet can be the topmost layer of the absorbent core, being substantially part of the core itself

The absorbent core can be any absorbent member which is generally compressible, conformable, non-irritating to the wearer's skin, and capable of absorbing and retaining liquids such as urine and other certain body exudates. The absorbent core may be manufactured in a wide variety of sizes and shapes (e.g:, rectangular, hourglass, T-shaped, asymmetric, etc) and from a wide variety of liquid-absorbent materials commonly used in disposable pull-on garments and other absorbent articles such as comminuted wood pulp which is generally referred to as airfelt. Examples of other suitable absorbent materials include creped cellulose wadding, meltblown polymers including coform, chemically stiffened, modified or cross-linked cellulosic fibers; tissue including tissue wraps and tissue laminates, absorbent foams; absorbent sponges, superabsorbent polymers; absorbent gelling materials; or any equivalent material or combinations of materials. The configuration and construction of the absorbent core may vary (e.g., the absorbent core may have varying caliper zones, a hydrophilic gradient, a superabsorbent gradient, or lower average density and lower average basis weight acquisition zones; or may include one or more layers or structures) Further, the size and absorbent capacity of the absorbent core may also be varied to accommodate wearers ranging from infants through adults However, the total absorbent capacity of the absorbent core should be compatible with the design loading and the intended use of the disposable article.

The absorbent core may include other optional components. One such optional component is the core wrap, i e, a material, typically but not always a nonwoven material, which either partially or totally surrounds the core. Suitable core wrap materials include, but are not limited to, cellulose, hydrophilically modified nonwoven materials, perforated films and combinations thereof

The backsheet may be typicvally impervious to liquids (e g., urine or menses) and can be manufactured from a thin plastic film. In an alternative embodiment the backsheet permits vapours to escape from the disposable absorbent article; for example, a microporous polyethylene film can be used for the backsheet. One suitable material for the backsheet of the absorbent article of the present invention can be a liquid impervious thermoplastic film having a thickness of from about 0.012 mm to about 0.051 mm, for example including polyethylene or polypropylene The backsheet can have a basis weight of from about 5 g/m² to about 35 g/m² However, it should be noted that other flexible liquid impervious materials may be alternatively used as the backsheet Herein, "flexible" refers to materials which are compliant and which will readily conform to the general shape and contours of the wearer's body The backsheet is typically positioned adjacent the outer-facing side of the absorbent core, and can be joined thereto by any suitable attachment means known in the art. For example, the backsheet may be secured to the absorbent core by a uniform continuous layer of adhesive, a patterned layer of adhesive, or an array of separate lines, spirals, or spots of adhesive.

When the absorbent article of the present invention is an article for feminine hygiene like a sanitary napkin, a panty liner, or an article for light incontinence, it is typically used by being adhered to the crotch portion of an undergarment by means of an attachment means, typically a layer of pressure sensitive adhesive, usually referred to as the panty fastening adhesive, provided onto the garment facing side of the backsheet. Before use, the panty fastening adhesive is protected by a release layer releasably adhered thereto, which is removed by the user to expose the adhesive when the article is to be applied to the undergarment. As it is known in the art, the release layer may be for example a sheet of siliconized paper, or a wrapper sheet, typically made of a polymeric film, which may also provide a releasable unitary wrapper for the article

According to the present invention, the absorbent article further comprises an odour control system comprising first and second classes of odour control materials The first class of odour control materials comprises at least one material having a low volatility, and the second class of odour control materials comprises at least one material having a high volatility. In the context of the present invention, the volatility is expressed in terms of Kovat Index (K1) which, as it is known in the art, is defined as the selective retention of perfume raw materials (PRMs) onto chromatographic columns. The values of the Kovat Index are obtained with a chromatographic column DB-5, 30 m, 0.25 mm, 1.00 µm, available from Agilent Technologies Inc (formerly J&W Scientific), operating under the following conditions 50-300°C, 4°C/min, 12.0 psi, constant flow; or equivalent equipment working under same or equivalent conditions, as can be readily determined by the man skilled in the art

Although primarily defined for perfume raw materials (PRMs), a Kovat Index can be measured generally for liquid and also gaseous materials which are not necessarily perfume raw materials (PRM), and which can be comprised in the first and second class of odour control materials in the odour control system of the absorbent article of the present invention, as will be explained hereinafter. As known, a material's, or more specifically a PRM's polarity, molecular weight, vapour pressure, boiling point, and the stationary phase property determine the extent of retention, and hence of the Kovat Index. According to the present invention, the at least one low volatility material in the first class of odour control materials has a Kovat Index higher than 1500, or between 1550 and 1900, and the at least one high volatility material in the second class of odour control materials has a Kovat Index of 1500 or less, or between 900 and 1500, or also between 1000 and 1400.

At least the second class of the odour control system of the absorbent article of the present invention is provided onto the garment facing side of the absorbent core, adjacent to the body facing side of the backsheet, on a surface area of at least 900 mm², or of 900 mm² to 2000 mm², or also of 900 mm² to 1300 mm². Alternatively, at least the second class of odour control materials is provided onto the garment facing side of the absorbent core, adjacent to the body facing side of the backsheet, on a surface area such that the ratio of this surface area to the core surface area is at least 0.08, or between 0 08 and 0.2, or also between 0.08 and 0 1, wherein the core surface area is defined as the surface area of the garment facing side of the absorbent core element. By saying "adjacent", it is meant that the odour control system, applied onto the garment facing side of the absorbent core, is located in the finished product substantially towards the body facing side of the backsheet In an embodiment of the present invention, the odour control system is in direct contact with the body facing side of the backsheet, i e, without any further layer interposed between the absorbent core and the backsheet. However, "adjacent" also means that an intermediate layer can be interposed between the garment facing side of the core, where the odour control system is applied, and the body facing side of the backsheet, for example a core wrap, or a tissue layer, e.g. a distribution layer An optional adhesive means between the core and the backsheet is not typically considered as a further layer in the context of the present invention.

The second class of odour control materials can be applied according to any suitable method to the garment facing side of the absorbent core of the absorbent article of the present invention Typical materials of the second class of odour control materials are liquid at room temperature, as will become apparent in more details further on, and therefore can be applied with known means to the garment facing side of the absorbent core, for example in drops, in any desired regular or irregular pattern. In an embodiment of the present invention, the second class of odour control materials of the odour control system is provided in one or more longitudinal stripes, for example in two longitudinal stripes parallel to each other and to a longitudinal symmetry axis of the absorbent article, and running symmetrically along substantially the entire core length at both sides of this longitudinal axis.

According to an embodiment of the present invention, at least the second class of odour control materials of the odour control system is provided onto the garment facing side of the core in a basis weight of 5 g/m² to 100 g/m², or of 10 g/m² to 80 g/m², or also of 20 g/m² to 60 g/m².

According to an embodiment of the present invention, the first class of odour control materials typically comprises materials which act on malodours of the exudates or fluids, or other malodorous substances which are present in the absorbent article during its use, while the second class of odour control materials typically comprises substances which act on the user's nose receptors.

Without being bound to any theory, it is believed that an odour control system according to the present invention and comprising a first class of odour control materials typically having a low volatility, and a second class of odour control materials typically having a high volatility, when applied on the garment facing side of the core on the selected surface area and optionally in the selected basis weight, being adjacent to the body facing side of the backsheet, or in an embodiment in direct contact thereto, i.e. with no intermediate layer interposed, such as a core wrapper, causes the more volatile material or materials of the odour control system, typically that or those in the second class of odour control materials, to diffuse through the backsheet layer itself, which is typically a polymeric film, usually a polyethylene film having a thickness in the range of 10-40 µm, e.g. about 20 µm The more volatile material or materials are however prevented from escaping outside of the absorbent article at least in part by the resistance to diffusion opposed by the backsheet material itself, and particularly by the layer of panty fastening adhesive provided on the garment facing side of the backsheet of the typical absorbent articles of the present invention, namely feminine hygiene articles such as sanitary napkins, panty liners, and articles for light adult incontinence, and by the release layer applied thereto At the same time, the more volatile material or materials of the odour control system have a minor tendency to escape from the topsheet of the absorbent article, owing to the greater thickness of the absorbent article layers and materials overlying the odour control system in the direction of the body facing side of the article. When the user takes the article from its packaging, typically exposes the panty fastening adhesive by removing the release layer in order to apply the article onto the panty, and thereby "freeing" the volatile odour control material or materials entrapped by the panty fastening adhesive and the release layer. The more volatile odour control material or materials therefore may act immediately after the article is readied for use by the user, by typically affecting the user's nose receptors even before the article is actually worn. The more volatile odour control material or materials, together with the less volatile odour control material or materials in the odour control system of the absorbent article of the present invention will also continue to perform their action, respectively towards the user's nose receptors and towards malodours of the exudates or other malodorous material in the absorbent article itself in use The absorbent article of the present invention, therefore, is capable of providing an improved and prolonged two-step odour control action, owing to the composition of the odour control system and to its particular positioning within the absorbent article, as described above.

### Odour control materials acting on the malodours or malodorous substance

The first class of odour control materials in the odour control system of the absorbent article of the present invention comprises at least a material having a Kovat Index higher than 1500, or between 1550 and 1900, hence with a low volatility The materials of the first class of odour control materials can be selected among known materials which counteract malodours

There are many materials known in the an for counteracting malodours in absorbent articles. Examples can be found in the references cited herein before Typical substances are zeolites, starch, activated carbon, cyclodextrine, chitin or chitosan and esters In general, as it is readily apparent to the man skilled in the art, solid materials such as those mentioned above do not actually have a Kovat Index, which can be only measured for liquid or also gaseous materials. However, solid materials can be said to be substantially non volatile, and in the context of the present invention can be considered to practically satisfy the prescribed condition for the Kovat Index of the at least one low volatility material in the first class of odour control materials in the odour control system according to the present invention

These actives can reduce the malodour unpleasantness according to different mechanisms, e.g. they can reduce the amount of malodorous molecules through absorption/adsorption mechanisms and/or can react with the malodorous molecules transforming them into low volatile/non-odorous ones and/or can suppress malodorous molecules volatility and/or can prevent the malodour generation by inhibiting degradative processes caused by microorganisms metabolic activity.

For the odour control system of an embodiment of the present invention a specific selection of such materials can be desired. It has been found that silica gel, aldehydes and mesoporous zeolites are particularly useful.

Silica gel is a porous, amorphous form of silica (SiO₂) It is composed of a vast network of interconnected microscopic pores. As opposed to zeolites, silica gels have larger pores with a wide range of diameters typically between 5 Å and 3000 Å Silica gel, which has proven particularly useful in the odour control system herein is the narrow silica gel with a pH of less than 7 Indeed it was discovered that this type of silica gel is effective in reducing the malodour level according to two different types of odour control mechanisms, absorption/adsorption of malodorous molecules on silica surface and neutralization of aminic components. The latter are a main source of malodour especially in feminine hygienic products. Silica gels with a pH of less than 6 may be desirable for certain embodiments.

It is possible to adjust the silica gel pore size range in the manufacturing process: Silica gels synthesized with an average pore size of about 10-50 Å are known as "narrow" pore silica gels; silica gels with an average pore size of about 110 Å and beyond are called "wide" pore silica gels. Silica gels with wide pore are generally more expensive than narrow silica gel In certain embodiments, the silica gel used herein is the narrow silica gel with average pore size of from 20- 40 Å, or in some embodiments 30 Å.

One suitable silica gel for the odour control system of the absorbent article of the present invention herein has a total surface area higher than 500 m²/g. The total surface area can be established by using the BET (Brunauer-Emmett-Teller) test. This test is based on the adsorption of nitrogen gas at 77°K onto the surfaces of the silica gel particles, i e also their internal cavities. The adsorbed volume of nitrogen is then established by comparing the nitrogen pressure before and after the adsorption Exemplary suitable silica gel materials include silica gel code 122 and 123 available from Grace, Columbia, MD, USA.

It has further been found by the present inventors that narrow silica gel or zeolites, especially mesoporous zeolites, can be used to stabilize the volatile odour control materials acting externally in the absorbent article Mesoporous zeolites are those zeolites with pore size from 20 to 500 Å. As indicated above, silica gel can absorb volatile substances and thus reduces its migration out of the absorbent article. Advantageously the shelf life of the absorbent articles is significantly prolonged thereby.

Without being bound by theory it is assumed that narrow silica gel can adsorb the most volatile and active compounds due to its porosity, particularly by creation of hydrogen bonding, and easily release active compounds during usage of product, i.e. due to presence of water that competes with the absorbed molecules in the formation of hydrogen bonds with the silica gel surface

The stabilization effect has been proven by running a Thermogravimetric analysis (TGA) on samples of narrow silica gel treated with 10 weight-% of menthyl acetate. Specifically, the method is based on evaluation of weight loss over time at specific temperature (40°C) The samples were kept at 40°C for 184 minutes. In order to have a basis for comparison the TGA analysis was performed on a sample of pure menthyl acetate and a sample of pure silica gel as references together with a sample of menthyl acetate (10%) + silica gel (ex Grace, coded 123). The results are listed in table 1 and illustrate that the sample with silica gel and MA has a significant lower weight loss than the samples of menthyl acetate alone

**Table 1**

| Data point | Sample composition | Weight Loss (%) |
|---|---|---|
| 1 | Menthyl acetate | ~100% |
| 2 | Silica gel + Menthyl acetate | 3 |
| 3 | Silica gel | 3 |

One component of body fluid malodour is ammonia. For example ammonia is present in high amounts in products used for urine absorption due to degradation of urea. Ammonia and its derivatives can react with aldehyde to form imines (according to the so-called Schiff base reaction).

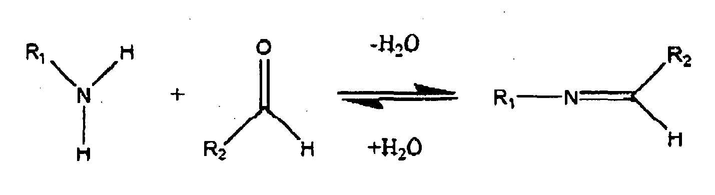

This reaction is catalyzed by enzymes and/or by a slightly-acidic pH 4 to 5. The moderate acid requirement is necessary to allow protonation of the hydroxyl intermediate to allow water to leave.

Unfortunately, most aldehydes capable of imine reaction have an unpleasant and/or too intense odour that can be disturbing to human nose and/or they are very volatile and so not stable on the product Therefore, it is desirable to select suitable materials for controlling malodour. Examples of suitable aldehydes for controlling malodour are those aldehydes that are able to react with aminic compounds according to Schiff base reaction and have not unpleasant odour. Suitable aldehydes include hexyl cinnamic aldehyde, alpha-amylcinnamic aldehyde, p-anisaldehyde, benzaldehyde, cinnamic aldehyde, cuminic aldehyde, decanal, p-t-butyl-alpha-methyldihydrocinnamaldehyde, 4-hydroxy-3-methoxycinnamaldehyde, 2-phenyl-3-(2-furyl)prop-2-enal, vanillin isobutyrate, ethyl vanillin acetate, vanillin acetate, cyclamen aldehyde, heptane lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-inmethoxybenzaldehyde, 3-hydroxybenzaldehyde, o-methoxycinnaldehyde, 3,5-dimethoxy-4-hydroxycinnamaldehyde, 2,8-dithianon-4-3n-4-carboxaldehyde, sorbinaldehyde, 2,4-heptadienal, 2,4-decadienal, 2,4-nonadienal, 2,4-nonadienal, (E,E)-,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial and mixtures thereof Particularly suitable aldehydes are hexyl cinnamic aldehyde, alpha-amylcinnamic aldehyde, decanal, 4-hydroxy-3-methoxycinnamaldehyde, 3,5-dimethoxy-4-hydroxycinnamaldehyde, 2-phenyl-3-(2-furyl)prop-2-enal, ethyl vanillin acetate, vanillin isobutyrate, vanillin acetate, asaronaldehyde. Some of the most desirable aldehydes for application herein are hexyl cunnamic aldehyde, alpha-amylcinnamaldehyde, 4-hydroxy-3-methoxycinnamaldehyde, and decanal

### Odour control materials acting on nose receptors

The second class of odour control materials in the odour control system of the absorbent article of the present invention comprises at least a material having a Kovat Index of 1550 or less, or between 900 and 1500, or also between 1000 and 1400, hence with a high volatility In an embodiment of the present invention, all odour control materials of the second class have the prescribed high volatility expressed in terms of the Kovat Index. The materials of the second class of odour control materials can be selected among known materials which act on the nose receptors of the user, e.g. can be perfumes or fragrances, which emanate a pleasant scent.

The second class of odour control materials in the odour control systems hence counteracts odours externally, outside the absorbent articles According to an embodiment of the present invention, suitable materials of the second class of odour control materials are those listed hereinafter which inhibit the receptors of the nose, hereinafter called "nose blocking". When used, these materials may significantly reduce the capability for the nose to detect the malodours. The action on the nose receptors, preferably the nose blocking with the selected materials according to this embodiment of the present invention, is possible due to the volatile nature of the materials selected, represented by the selected Kovat Index, which are evaporating out of the absorbent article according to the mechanism described above, and are then inhaled into the nose of an individual generally within somewhat close range of the article, e.g. within about 0 to 10 meters of the article (although this should in no way be intended to limit the scope of the invention) by normal breathing The blocking of the nose receptors is of course only temporary.

Suitable nose blocking materials include menthol, menthyl acetate, 3-buten-2-one,3-methyl-4-(2,6,6-trimethyl-2-cyclohexen-1-yl)-, 4-(2,6,6-trimethylcyclohen-1-en-1-yl)but-3-en-2-one, 3-buten-2-one,4-(2,6,6-trimethyl-2-cyclohexen-1-yl)-, (E)-, menthyl lactate, isomenthyl acetate, isomenthyl propionate, isomenthyl isobutyrate, isomenthyl propionate, isomenthyl butyrate, camphor and p-menthane. These materials also include their isomeric forms, diastereomers and enantiomers. Advantageously, in general, the above materials have only a very slight inherent odour but show a high degree of nose receptor blocking

According to an embodiment of the present invention, an absorbent article comprising topsheet, backsheet and absorbent core can further comprise an odour control system comprising a first class of odour control materials selected from the group consisting of silica gel having a pH of less than 7, mesoporous zeolites having pores sizes of from 20 to 500 Å and mixtures thereof, and aldehydes in turn selected from the group as described above, and a second class of odour control materials selected from the group consisting of nose blocking materials as described above, wherein the at least second class of odour control materials of the odour control system is provided onto the garment facing side of the absorbent core, being adjacent to the body facing side of the backsheet, on a surface area of at least 900 mm², or of 900 mm² to 2000 mm², or also of 900 mm² to 1300 mm². Alternatively, the at least second class of odour control materials is provided onto the garment facing side of the absorbent core, being adjacent to the body facing side of the backsheet, on a surface area such that the ratio of said surface area to said core surface area is at least 0 08, or between 0.08 and 0.2, or also between 0.08 and 0.1. According to the above embodiment, any combinations with all other additional or alternative features described herein are also comprised

According to an alternative embodiment of the present invention, the high volatility materials of the second class of odour control materials can be selected among materials having a pleasant odour. According to the law of Dalton, due to their high partial pressure in the headspace of the absorbent article, and also owing to their application in selected position, areas and preferred basis weights as explained before, such highly volatile components are believed to reduce the molar fraction of the malodorous compounds having a lower partial pressure, and provide a pleasant odour to the user. Suitable highly volatile components include materials that have a KI (Kovat Index) below 1500.

Suitable highly volatile components that act according to this mechanism include e.g. limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, di-hydrocumarin, di-hydromyrcenyl acetate, geraniol, geranial, isoamylacetate, ethyl, and /or methyl acetate, para-cresol and para-cymene

According to a further embodiment of the present invention, the above high volatility materials having a pleasant odour are included in the second class of odour control materials of the odour control system in the absorbent article of the present invention in addition to the "nose blocking" materials described above.

### Optional further components

### a)Hydrogel forming absorbent polymers

The absorbent article of the present invention can also comprise, in addition to the odour control system, a hydrogel forming absorbent polymer Hydrogel forming absorbent polymers useful in the present invention include a variety of water-insoluble, but water-swellable polymers capable of absorbing large quantities of fluids, and are also commonly referred to in the art as absorbent gelling materials, or superabsorbent materials Absorbent gelling materials, as it is known in the art, are widely used to provide absorbent articles with improved absorbent capacity, being typically comprised in absorbent structures, alone or in combination with fibrous absorbent material such as for example pulp, airfelt, tissue or nonwoven layers. Absorbent gelling materials are also used in combination with known odour control materials, as they can provide additional benefit in odour control capability together with their liquid absorption capacity.

Any absorbent gelling materials which are known in the art can be comprised in the absorbent article of the present invention, in addition to the odour control system. Suitable absorbent gelling materials can include hydrolyzed acrylonitrile grafted starch, acrylic acid grafted starch, polyacrylates, maleic anhydride-based copolymers and combinations thereof Particularly suitable absorbent gelling materials are the polyacrylates and acrylic acid grafted starch.

The absorbent gelling materials herein before described are typically used in the form of discrete particles. Such absorbent gelling materials can be of any desired shape, e g, spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used

The size of the absorbent gelling material panicles may vary over a wide range. For example, particle sizes comprised between 150 µ and 800 µ are commonly used in the an, although smaller or greater particle sizes can be also used "Particle Size" as used herein means the weighted average of the smallest dimension of the individual particles The absorbent gelling materials are incorporated into the absorbent article of the present invention according to known means, typically within the absorbent core.

### b) Solvents

Further additional ingredients include solvents as carriers for incorporating the odour control materials into the absorbent article. Suitable solvents are e.g. benzyl-benzoate, isopropyl myristate, methyl abietate, ethanol, isopropanol, diethylene glycol monoethyl ether, glycerol; propylene glycol, 1,2-butylene glycol, dipropylene glycol, 2-methyl-2,4-pentanediol, diethyl phthalate, trietyl citrate, diethyl sebacate.

### The odour control system

It has been found by the present inventors that the combination of certain, separately known odour control materials results in a synergistic effect in terms of odour reduction Specifically, in an embodiment of the present invention a synergistic effect in terms of malodour reduction is observed when combining odour control materials acting on the malodours or malodorous substance itself and odour control materials acting on nose receptors, as described above. In fact, an odour control system combining actives of the aforementioned two classes may reduce the malodours more efficiently than the mathematical combination of each material acting individually

Without wishing to be bound by theory this is believed to be a result of the neutralization of the malodorous molecules in the absorbent article by the odour control materials acting internally, which reduces the concentration of these molecules in the head space and thus, in the air Due to this, and in combination with the selected positioning in the absorbent article of the present invention, as explained above, the odour control materials acting externally onto the nose receptors can exploit their maximum activity.

When the odour control system of the present invention is used in absorbent articles the individual odour control materials can be employed at variable amounts. When considering the specific suitable odour control materials of the first class, namely odour control materials acting internally, for silica gel an amount of from 5 g/m² to 300 g/m², or from 20 g/m² to 100 g/m² has proven useful. For aldehydes, an amount of from 0 05 g/m² to 20 g/m², or from 0 5 g/m² to 5 g/m² has proven useful An example of a suitable odour control material of the second class, namely a material acting on nose receptors, can include menthyl acetate in the range from 0 05 g/m² to 20 g/m², or from 0.5 g/m² to 5 g/m²

The odour control system can comprise the first class of odour control materials, for example typically acting on the malodours or malodorous substance, in relation to the second class of odour control materials, for example typically acting on the nose receptors, at a ratio of from 501 to 1:50 by weight, or from 30.1 to 1.30 by weight or also from 1.15 to 15:1 by weight. In one embodiment, the odour control system can comprise silica gel and menthyl acetate at a ratio of 15:1 by weight In an alternative embodiment the odour control system can comprise alpha-amylcinnamaldehyde and menthyl acetate at a ratio of 1:1 by weight.

### Absorbent article

The absorbent article being provided with the odour control system herein can be any kind of absorbent article of personal hygiene known in the art Particularly suitable are absorbent articles for personal feminine hygiene such as sanitary napkins, pantiliners and articles for light adult incontinence. The odour control system of the present invention can be present in any part of the absorbent article, provided at least the second class of odour control material is applied onto the garment facing side of the absorbent core in the prescribed surface area. According to the present invention the odour control system can either be present in the absorbent article as an intimate mixture of the at least two classes of odour control materials or with both classes of odour control materials being separate from each other. For example, an embodiment of the present invention can have the first class of odour control materials (acting on the malodours or malodorous substance) placed on the wearer-facing side of the absorbent core, or alternatively within the structure of the absorbent core, while the second class of odour control materials (acting on nose receptors) can be placed on the garment-facing side of the absorbent core. Taking into account the stabilization described herein before, one execution of this embodiment can be an absorbent article being provided with silica gel on the wearer-facing side of the absorbent core and with menthyl acetate on the garment-facing side of the absorbent core However, other arrangements of the first and second odour control materials are contemplated.

### Test methods and data

for proving The synergistic effect on odour reduction of an odour control system according to an embodiment of the present invention several test samples have been prepared, which are all being exposed to a 0.1% aqueous solution of ammonia, which serves as a test malodorous substance The first data point in table 2 serves as a benchmark as it represents the degree of unpleasantness of the ammonia solution without added odour control material Data points 2-3 in table 2 are illustrating the malodour-reducing activity of two exemplary odour control materials alone, specifically alpha-amylcinnamaldehyde as exemplary aldehyde and menthyl acetate as exemplary odour control material ("nose blocking" type) acting on the nose receptors

The last data point 4 in table 2 is illustrating the activity of the odour control system of this embodiment of the present invention For comparability two individual odour control materials were mixed at half their amount compared to data points 2-3. Data point 4 has been obtained by testing a mixture of alpha-amylcinnamaldehyde and menthyl acetate It is clearly obtainable from data point 4 that the odour reduction performance of the odour control system is significantly better than the one of the individual odour control materials. Thus, synergistic odour control activity has been proven.

**Table 2**

| Data point # | Sample composition | Odor unpleasantness (%) |
|---|---|---|
| 1 | Malodorant solution (10 ml) | **100** |
| 2 | Alpha-Amylcinnamaldehyde (16 mg) + Malodorant solution (10 ml) | **64** |
| 3 | Menthyl acetate (16 mg) + Malodorant solution (10 ml) | **46** |
| 4 | Alpha-Amylcinnamaldehyde (8mg) + Menthyl acetate (8 mg) + Malodorant solution (10 ml) | **30** |

The odour unpleasantness and pleasantness of these samples have been evaluated by a panel of expert graders In particular five different expert graders have evaluated 4 replicates for each sample Odour unpleasantness have been evaluated by using a scale from -10 to + 0, where - 10 indicates the max odour unpleasantness, 0 indicates no odour. Data have been then reported as % relative unpleasantness vs. Reference (malodourant solution). The odour evaluation has been performed in adequate room, at controlled temperature T (25°C) The room is equipped with appropriate conditioning system allowing continuous exchange of air The samples were held in numbered metal Trays, which were covered with aluminium foil between the actual gradings.

Each dimension for which a value is defined herein is a technical dimension, which, in the context of the present invention is not to be understood literal. Hence, all embodiments having dimensions functionally equivalent to the dimensions stated herein are intended to be covered by the scope of the invention, e.g. a dimension of "40 mm" has to be understood as meaning "about 40 mm".

## Claims

1. An absorbent article comprising a liquid permeable topsheet, a backsheet, and an absorbent core comprised between said topsheet and said backsheet, each of said topsheet, backsheet and absorbent core having a body facing side and a garment facing side, said garment facing side of said core having a core surface area, said absorbent article comprising an odour control system comprising first and second classes of odour control materials, wherein said first class comprises at least a material having a Kovat Index (KI) higher than 1500, preferably between 1550 and 1900, and said second class comprises at least a material having a Kovat index of 1500 or less, preferably between 900 and 1500, more preferably between 1000 and 1400,
at least said second class of odour control materials of said odour control system being provided onto said garment facing side of said absorbent core, and being adjacent to the body facing side of said backsheet, on a surface area of at least 900 mm², preferably of 900 mm² to 2000 mm², more preferably of 900 mm² to 1300 mm², or on a surface area such that the ratio of said surface area to said core surface area is at least 0 08, preferably between 0 08 and 0.2, more preferably between 0.08 and 0 1.

2. An absorbent article according to claim 1, wherein at least said second class of odour control materials of said odour control system is provided in a basis weight of 5 g/m² to 100 g/m², preferably of 10 g/m² to 80 g/m², more preferably of 20 g/m² and 60 g/m².

3. An absorbent article according to any preceding claim, wherein at least said second class of odour control materials of said odour control system provided onto said garment facing side of said absorbent core is in direct contact with the body facing side of said backsheet

4. An absorbent article according to any preceding claim, wherein at least said second class of odour control materials of said odour control system is provided onto said garment facing side of said absorbent core in one or more longitudinal stripes.

5. An absorbent article according to any preceding claim, wherein said first class of odour control materials reduces odour by acting on malodours or a malodorous substance in the absorbent article and said second class of odour control material reduces odour by acting on the user's nose receptors, wherein said first class of odour control materials is selected from the group consisting of silica gel having a pH of less than 7, aldehydes, mesoporous zeolites having pores sizes of from 20 to 500 Å and mixtures thereof, wherein said aldehydes are selected from the group consisting of hexyl cinnamic aldehyde, alpha-amylcinnamic aldehyde, p-anisaldehyde, benzaldehyde, cinnamic aldehyde, cuminic aldehyde, decanal, cyclamen aldehyde, p-t-butyl-alpha-methyldihydrocinnamaldehyde, 4-hydroxy-3-methoxycinnamaldehyde, vanillin isobutyrate, 2-phenyl-3-(2-furyl)prop-2-enal, ethyl vanillin acetate, vanillin acetate, heptanal, lauryl aldehyde, nonanal, octanal, phenylacetaldehyde, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2,3,4-trimethoxybenzaldehyde, protocatechualdehyde, heliotropine, 4-ethoxy-3-methoxy benzaldehyde, 3,4,5-trimethoxybenzaldehyde, 3-hydroxybenzaldehyde, o-methoxycinnamaldehyde, 3,5-dimethoxy-4-hydroxycinnamaldehyde, 2,8-dithianon-4-3n-4-carboxoldehyde, sorbinaldehyde, 2,4-heptadienal, 2,4-decadienal, 2,4-nonadienal, 2,4-nonadienal, (E,E)- ,2,4-octadien-1-al, 2,4-octadienal, 2,4-dodecadienal, 2,4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, pyruvaldehyde, ethanedial and mixtures thereof and said second class of odour control materials is selected from the group consisting of menthol, menthyl acetate, menthyl lactate, 3-buten-2-one,3-methyl-4-(2,6,6-trimethyl-2-cyclohexen-1-yl)-, 4-(2,6,6-trimethylcyclohen-1-en-1-yl)but-3-en-2-one, 3-buten-2-one,4-(2,6,6-trimethyl-2-cyclohexen-1-yl)-,(E)- isomenthyl acetate, isomenthyl propionate, isomenthyl isobutyrate, isomenthyl propionate, isomenthyl butyrate, camphor, p-menthane and mixtures thereof.

6. The absorbent article of any preceding claim, wherein each material in said second class of odour control materials has a Kovat Index of 1500 or less.

7. The absorbent article of any of the previous claims, wherein the odour control system further comprises a highly volatile component selected from the group consisting of limonene, eucalyptol, cresol, linalool, tetra-hydrolinalool, myrcenol, tetra hydromyrcenol, di-hydromyrcenol, myrcene, cytronellol, cytronellyil derivatives, geraniol, geranyl derivatives, linalyl acetate, mugetanol, eugenol, jasmal, terpineol, pinanol, cedrene, damascone, beta pinene, cineole and its derivatives, nonadienol, ethylhexanal, octanol acetate, methyl furfural, terpinene, thujene, amylacetate, benzylacetate, camphene, citronellal, dihydrocumarin, dy hydromyrcenyl acetate, geraniol, geranial, isoamylacetate, ethyl, and/or triethyl acetate, para-cresol, para-cymene, methyl abietate and mixtures thereof

8. The absorbent article of any of claims 5 to 7, wherein the odour control system comprises narrow silica gel in an amount of from 5 g/m² to 300 g/m², preferably from 20 g/m² to 100 g/m².

9. The absorbent article of any of claims 5 to 8, wherein the odour control system comprises said aldehyde or aldehydes in an amount of from 0 05 g/m² to 20 g/m², preferably from 0 5g/m² to 5 g/m²

10. The absorbent article of any of claims 5 to 9, wherein the odour control system comprises hexyl cinnamic aldehyde

11. The absorbent article of any of claims 5 to 10, wherein the odour control system comprises menthyl acetate in an amount of from 0 05 g/m² to 20 g/m² preferably from 0.5 g/m² to 5 g/m²

12. The absorbent article of any of the previous claims, wherein the odour control system comprises said first class of odour control materials in relation to said second class of odour control materials at a ratio of from 50·1 to 1.50 by weight, preferably from 30·1 to 1.30 by weight, and more preferably from 1·15 to 15·1 by weight

13. The absorbent article of claim 12, wherein the odour control system comprises narrow silica gel and menthyl acetate at a ratio of 15.1 by weight

14. The absorbent article of claim 12, wherein the odour control system comprises hexyl cinnamic aldehyde and menthyl acetate at a ratio of 1·1 by weight.

15. The absorbent article of any preceding claim, said absorbent article being a sanitary napkin, a pantiliner, or a light adult incontinence product, and further comprising an adhesive means on said garment facing side of said backsheet, and a release layer applied thereto

## Patentansprüche

1. Absorptionsartikel, der eine flüssigkeitsdurchlässige Oberschicht, eine Unterschicht und einen zwischen der Oberschicht und der Unterschicht angeordneten Absorptionskern umfasst, wobei die Oberschicht, Unterschicht und der Absorptionskern jeweils eine zum Körper weisende Seite und eine zum Kleidungsstück weisende Seite aufweisen, wobei die zum Kleidungsstück weisende Seite des Kerns einen Kernoberflächenbereich aufweist, wobei der Absorptionsartikel ein Geruchsregulierungssystem aufweist, das eine erste und eine zweite Klasse an Geruchsregulierungsmaterialien umfasst, wobei die erste Klasse zumindest ein Material mit einem Kovats-Index (KI) von über 1500 aufweist, vorzugsweise zwischen 1550 und 1900, und die zweite Klasse zumindest ein Material mit einem Kovats-Index von 1500 oder weniger umfasst, vorzugsweise zwischen 900 und 1500, mehr bevorzugt zwischen 1000 und 1400,
wobei zumindest die zweite Klasse an Geruchsregulierungsmaterialien des Geruchsregulierungssystems auf der zum Kleidungsstück weisenden Seite des Absorptionskerns bereitgestellt wird und an die zum Körper weisende Seite der Unterschicht angrenzt, und zwar auf einem Oberflächenbereich von mindestens 900 mm², vorzugsweise 900 mm² bis 2000 mm², mehr bevorzugt 900 mm² bis 1300 mm², oder auf einem solchen Oberflächenbereich, dass das Verhältnis zwischen Oberflächenbereich zu Kernoberflächenbereich mindestens 0,08 beträgt, vorzugsweise zwischen 0,08 und 0,2, mehr bevorzugt zwischen 0,08 und 0,1.

2. Absorptionsartikel nach Anspruch 1, wobei zumindest die zweite Klasse an Geruchsregulierungsmaterialien des Geruchsregulierungssystems mit einem Grundgewicht von 5 g/m² bis 100 g/m² vorliegt, vorzugsweise von 10 g/m² bis 80 g/m², mehr bevorzugt von 20 g/m² und 60 g/m².

3. Absorptionsartikel nach einem der vorgenannten Ansprüche, wobei zumindest die zweite Klasse an Geruchsregulierungsmaterialien des Geruchsregulierungssystems, das auf der zum Kleidungsstück weisenden Seite des Absorptionskerns vorhanden ist, in direktem Kontakt mit der zum Körper weisenden Seite der Unterschicht steht.

4. Absorptionsartikel nach einem der vorgenannten Ansprüche, wobei zumindest die zweite Klasse an Geruchsregulierungsmaterialien des Geruchsregulierungssystems, das auf der zum Kleidungsstück weisenden Seite des Absorptionskerns vorhanden ist, als ein oder mehrere Längsstreifen vorliegt.

5. Absorptionsartikel nach einem der vorgenannten Ansprüche, wobei die erste Klasse an Geruchsregulierungsmaterialien durch Wirkung auf schlechte Gerüche oder eine schlecht riechende Substanz im Absorptionsartikel Geruch reduziert und die zweite Klasse an Geruchsregulierungsmaterial durch Wirkung auf die Nasenrezeptoren eines Benutzers Geruch reduziert, wobei die erste Klasse an Geruchsregulierungsmaterialien aus der Gruppe bestehend aus Silicagel mit einem pH-Wert von unter 7, Aldehyden, mesoporösen Zeolithen mit einer Porengröße von 20 bis 500 Å; und Mischungen davon ausgewählt ist, wobei die Aldehyde aus der Gruppe bestehend aus Hexylzimtaldehyd, alpha-Amylzimtaldehyd, p-Anisaldehyd, Benzaldehyd, Zimtaldehyd, Cuminaldehyd, Decanäl, Cyclamenaldehyd, p-t-Butyl-alpha-methyldihydrozimtaldehyd, 4-Hydroxy-3-methoxyzimtaldehyd, Vanillinisobutyrat, 2-Phenyl-3-(2-furyl)prop-2-enal, Ethylvanillinacetat, Vanillinacetat, Heptanal, Laurylaldehyd, Nonanal, Octanal, Phenylacetaldehyd, Phenylpropylaldehyd, Vanillin, Salycilaldehyd, Cytral, 2,4-Dihydroxy-3-methyl-benzaldehyd, 2-Hydroxy-4-methylbenzaldehyd, 5-Methylsalicylaldehyd, 4-Nitrobenzaldehyd, o-Nitrobenzaldehyd, 5-Ethyl-2-thiophencarbaldehyd, 5-Methyl-2-thiophencarboxaldehyd, 2-Thiophencarbaldehyd, Asaronaldehyd, 5-(Hydroxymethyl)-2-furaldehyd, 2-Benzofurancarboxaldehyd, 2,3,4-Trimethoxybenzaldehyd, Protocatechualdehyd, Heliotropin, 4-Ethoxy-3-methoxybenzaldehyd, 3,4,5-Trimethoxybenzaldehyd, 3-Hydroxybenzaldehyd, o-Methoxyzimtaldehyd, 3,5-Dimethoxy-4-hydroxyzimtaldehyd, 2,8-Dithianon-4-3n-4-carboxaldehyd, Sorbinaldehyd, 2,4-Heptadienal, 2,4-Decadienal, 2,4-Nonadienal, 2,4-Nonadienal, (E,E)- ,2,4-Octadien-1-al, 2,4-Octadienal, 2,4-Dodecadienal, 2,4-Undecadienal, 2,4-Tridecadien-1-al, 2-Trans-4-cis-7-cis-tridecatrienal, P-iperonylidenpropionaldehyd, 2-Methyl-3-(2-furyl)acrolein, 2,4-Pentadienal, 2-Furfurylidenbutyraldehyd, 3-(2-Furyl)acrolein, Pyruvaldehyd, Ethandial und Mischungen davon ausgesucht ist,
und die zweite Klasse an Geruchsregulierungsmaterialien aus der Gruppe bestehend aus Menthol, Menthylacetat, Menthyllactat, 3-Buten-2-on, 3-Methyl-4-(2,6,6-trimethyl-2-cyclohexen-1-yl)-, 4-(2,6,6-Trimethylcyclohen-1-en-1-yl)but-3-en-2-on, 3-Buten-2-on, 4-(2,6,6-Trimethyl-2-cyclohexen-1-yl)-, (E)- Isomenthylacetat, Isomenthylpropionat, Isomenthylisobutyrat, Isomenthylpropionat, Isomenthylbutyrat, Kampfer, p-Menthan und Mischungen davon ausgesucht ist.

6. Absorptionsartikel nach einem der vorgenannten Ansprüche, wobei jedes Material der zweiten Klasse an Geruchsregulierungsmaterialien einen Kovats-Index von 1500 oder weniger aufweist.

7. Der Absorptionsartikel nach einem der vorgenannten Ansprüche, wobei das Geruchsregulierungssystem des Weiteren einen stark flüchtigen Bestandteil ausgewählt aus der Gruppe bestehend aus Limonen, Eucalyptol, Cresol, Linalool, Tetrahydrolinalool, Myrcenol, Tetrahydromyrcenol, Dihydromyrcenol, Myrcen, Cytronellol, Cytronellyilderivaten, Geraniol, Geranylderivaten, Linalylacetat, Mugetanol, Eugenol, Jasmal, Terpineol, Pinanol, Cedren, Damascon, Betapinen, Cineol und seinen Derivaten, Nonadienol, Ethylhexanal, Octanolacetat, Methylfurfural, Terpinen, Thujen, Amylacetat, Benzylacetat, Camphen, Citronellal, Dihydrocumarin, Dyhydromyrcenylacetat, Geraniol, Geranial, Isoamylacetat, Ethyl und/oder Triethylacetat, Paracresol, Paracymen, Methylabietat und Mischungen davon umfasst.

8. Absorptionsartikel nach einem der Ansprüche 5 bis 7, wobei das Geruchsregulierungssystem engporiges Silicagel in einem Anteil von 5 g/m² bis 300 g/m² umfasst, vorzugsweise von 20 g/m² bis 100 g/m².

9. Absorptionsartikel nach einem der Ansprüche 5 bis 8, wobei das Geruchsregulierungssystem das Aldehyd oder die Aldehyde in einem Anteil von 0,05 g/m² bis 20 g/m² umfasst, vorzugsweise von 0,5 g/m² bis 5 g/m².

10. Absorptionsartikel nach einem der Ansprüche 5 bis 9, wobei das Geruchsregulierungssystem Hexylzimtaldehyd umfasst.

11. Absorptionsartikel nach einem der Ansprüche 5 bis 10, wobei das Geruchsregulierungssystem Menthylacetat in einem Anteil von 0,05 g/m² bis 20 g/m² umfasst, vorzugsweise von 0,5 g/m² bis 5 g/m².

12. Absorptionsartikel nach einem der vorgenannten Ansprüche, wobei das Geruchsregulierungssystem die erste Klasse an Geruchsregulierungsmaterialien im Verhältnis zur zweiten Klasse an Geruchsregulierungsmaterialien in einem Gewichtsverhältnis von 50:1 bis 1:50 umfasst, vorzugsweise von 30:1 bis 1:30 und mehr bevorzugt von 1:15 bis 15:1.

13. Absorptionsartikel nach Anspruch 12, wobei das Geruchsregulierungssystem engporiges Silicagel und Menthylacetat in einem Gewichtsverhältnis von 15:1 umfasst.

14. Absorptionsartikel nach Anspruch 12, wobei das Geruchsregulierungssystem Hexylzimtaldehyd und Menthylacetat in einem Gewichtsverhältnis von 1:1 umfasst.

15. Absorptionsartikel nach einem der vorgenannten Ansprüche, wobei der Absorptionsartikel eine Damenbinde, eine Slipeinlage oder ein leichtes Inkontinenzprodukt für Erwachsene ist und des Weiteren ein Haftmittel auf der zum Kleidungsstück weisenden Seite der Unterschicht und eine darauf aufgebrachte Löseschicht umfasst.

## Revendications

1. Article absorbant comprenant une feuille de dessus perméables aux liquides, une feuille de fond, et une âme absorbante comprise entre ladite feuille de dessus et ladite feuille de fond, chacune desdites feuille de dessus, feuille de fond et âme absorbante ayant un côté faisant face au corps et un côté faisant face au vêtement, ledit côté faisant face au vêtement de ladite âme ayant une superficie d'âme, ledit article absorbant comprenant un système de régulation des odeurs comprenant des première et deuxième classes de matériaux de régulation des odeurs, dans lequel ladite première classe comprend au moins un matériau ayant un indice de Kovat (KI) supérieur à 1500, de préférence entre 1550 et 1900, et ladite deuxième classe comprend au moins un matériau ayant un indice de Kovat de 1500 ou moins, de préférence entre 900 et 1500, plus préférablement entre 1000 et 1400,
au moins ladite deuxième classe de matériaux de régulation des odeurs dudit système de régulation des odeurs fournie sur ledit côté faisant face au vêtement de ladite âme absorbante, et étant adjacente au côté faisant face au corps de ladite feuille de fond, sur une superficie d'au moins 900 mm², de préférence de 900 mm² à 2000 mm², plus préférablement de 900 mm² à 1300 mm², ou sur une superficie telle que le rapport de ladite superficie sur ladite superficie d'âme est d'au moins 0,08, de préférence entre 0,08 et 0,2, plus préférablement entre 0,08 et 0,1.

2. Article absorbant selon la revendication 1, dans lequel au moins ladite deuxième classe de matériaux de régulation des odeurs dudit système de régulation des odeurs est fournie dans une masse surfacique de 5 g/m² à 100 g/m², de préférence de 10 g/m² à 80 g/m², plus préférablement de 20 g/m² à 60 g/m².

3. Article absorbant selon l'une quelconque des revendications précédentes, dans lequel au moins ladite deuxième classe de matériaux de régulation des odeurs dudit système de régulation des odeurs fournie sur ledit côté faisant face au vêtement de ladite âme absorbante est en contact direct avec le côté faisant face au corps de ladite feuille de fond.

4. Article absorbant selon l'une quelconque des revendications précédentes, dans lequel au moins ladite deuxième classe de matériaux de régulation des odeurs dudit système de régulation des odeurs est fournie sur ledit côté faisant face au vêtement de ladite âme absorbante dans une ou plusieurs bandes longitudinales.

5. Article absorbant selon l'une quelconque des revendications précédentes, dans lequel ladite première classe de matériaux de régulation des odeurs réduit l'odeur en agissant sur les mauvaises odeurs ou une substance malodorante dans l'article absorbant et ladite deuxième classe de matériau de régulation des odeurs réduit l'odeur en agissant sur les récepteurs du nez de l'utilisateur, dans lequel ladite première classe de matériaux de régulation des odeurs est choisie dans le groupe constitué de gel de silice ayant un pH de moins de 7, des aldéhydes, des zéolites mésoporeuses ayant des grosseurs des pores allant de 20 à 500 Å; et leurs mélanges, dans lequel lesdits aldéhydes sont choisis dans le groupe constitué de l'aldéhyde hexyl-cinnamique, l'aldéhyde alpha-amylcinnamique, le p-anisaldéhyde, le benzaldéhyde, l'aldéhyde cinnamique, l'aldéhyde cuminique, le décanal, l'aldéhyde de cyclamen, le p-t-butyl-alpha-méthyldihydrocinnamaldéhyde, le 4-hydroxy-3-méthoxycinnamaldéhyde, l'isobutyrate de vanilline, le 2-phényl-3-(2-furyl)prop-2-énal, l'acétae phénylacétaldéhte d'éthyl-vanilline, l'acétate de vanilline, l'heptanal, l'aldéhyde laurylique, le nonanal, l'octanal, aldéhyde phénylacétique, le phényl-propyl-aldéhyde, la vanilline, l'aladéhyde salycilique, le cytral, le 2,4-dihydroxy-3-méthylbenzaldéhyde, le 2-hydroxy-4-méthylbenzaldéhyde, les aldéhydes 5-méthyl salicyliques, le 4-nitrobenzaldéhyde, l'o-nitrobenzaldéhyde, le 5-éthyl-2-thiophènecarbaldéhyde, le 5-méthyl-2-thiophènecarboxaldéhyde, le 2-thiophènecarbaldéhyde, l'asaronaldéhyde, le 5-(hydroxymethyl)-2-furaldéhyde, le 2-benzofurancarboxaldéhyde, le 2,3,4-triméthoxybenzaldéhyde, le protocatéchualdéhyde, l'héliotropine, le 4-éthoxy-3-méthoxy benzaldéhyde, le 3,4,5-triméthoxybenzaldéhyde, le 3-hydroxybenzaldéhyde, l'o-méthoxycinnamaldéhyde, le 3,5-diméthoxy-4-hydroxycinnamaldéhyde, le 2,8-dithianon-4-3n-4-carboxaldéhyde, le sorbinaldéhyde, le 2,4-heptadiénal, le 2,4-décadiénal, le 2,4-nonadiénal, le 2,4-nonadiénal, le (E,E)-,2,4-octadién-1-al, le 2,4-octadiénal, le 2,4-dodécadiénal, le 2,4-undécadiénal, le 2,4-tridécadién-1-al, le 2-trans-4-cis-7-cis-tridécatriénal, le pipéronylidène propionaldéhyde, la 2-méthyl-3-(2-furyl)acroléine, le 2,4-pentadiénal, le 2-furfurylidène butyraldéhyde, la 3-(2-furyl)acroléine, le pyruvaldéhyde, l'éthanedial et leurs mélanges,
et ladite deuxième classe de matériaux de régulation des odeurs est choisie dans le groupe constitué de menthol, acétate de menthyle, lactate de menthyle, 3-butén-2-one,3-méthyl-4-(2,6,6-triméthyl-2-cyclohexén-1-yl)-, 4-(2,6,6-triméthylcyclohén-1-én-1-yl)but-3-én-2-one, 3-butén-2-one,4-(2,6,6-triméthyl-2-cyclohexén-1-yl)-, acétate de (E)- isomenthyle, propionate d'isomenthyle, isobutyrate d'isomenthyle, propionate d'isomenthyle, butyrate d'isomenthyle, camphre, p-menthane et leurs mélanges.

6. Article absorbant selon l'une quelconque des revendications précédentes, dans lequel chaque matériau dans ladite deuxième classe de matériaux de régulation des odeurs a un indice de Kovat de 1500 ou moins.

7. Article absorbant selon l'une quelconque des revendications précédentes, dans lequel le système de régulation des odeurs comprend en outre un composant hautement volatil choisi dans le groupe constitué de limonène, eucalyptol, crésol, linalol, tétra-hydrolinalol, myrcénol, tétra-hydromyrcénol, di-hydromyrcénol, myrcène, cytronellol, dérivés de cytronellyile, géraniol, dérivés de géranyle, acétate de linalyle, mugétanol, eugénol, jasmal, terpinéol, pinanol, cédrène, damascone, bêta pinène, cinéole et ses dérivés, nonadiénol, ethylhexanal, acétate d'octanol, méthyl furfural, terpinène, thujène, amylacétate, benzylacétate, camphène, citronellal, dihydrocoumarine, acétate de dyhydromyrcényle, géraniol, géranial, isoamylacétate, acétate d'éthyle et/ou de triéthyle, para-crésol, para-cyinène, abiétate de méthyle et leurs mélanges.

8. Article absorbant selon l'une quelconque des revendications 5 à 7, dans lequel le système de régulation des odeurs comprend du gel de silice étroit en une quantité de 5 g/m² à 300 g/m², de préférence de 20 g/m² à 100 g/m².

9. Article absorbant selon l'une quelconque des revendications 5 à 8, dans lequel le système de régulation des odeurs comprend ledit aldéhyde ou lesdits aldéhydes en une quantité de 0,05 g/m² à 20 g/m², de préférence de 0,5 g/m² à 5 g/m².

10. Article absorbant selon l'une quelconque des revendications 5 à 9, dans lequel le système de régulation des odeurs comprend de l'aldéhyde hexyl-cinnamique.

11. Article absorbant selon l'une quelconque des revendications 5 à 10, dans lequel le système de régulation des odeurs comprend de l'acétate de menthyle en une quantité de 0,05 g/m² à 20 g/m², de préférence de 0,5 g/m² à 5 g/m².

12. Article absorbant selon l'une quelconque des revendications précédentes, dans lequel le système de régulation des odeurs comprend ladite première classe de matériaux de régulation des odeurs par rapport à ladite deuxième classe de matériaux de régulation des odeurs à un rapport allant de 50:1 à 1:50 en poids, de préférence de 30:1 à 1:30 en poids, et plus préférablement de 1:15 à 15:1 en poids.

13. Article absorbant selon la revendication 12, dans lequel le système de régulation des odeurs comprend du gel de silice étroit et de l'acétate de menthyle à un rapport de 15:1 en poids.

14. Article absorbant selon la revendication 12, dans lequel le système de régulation des odeurs comprend de l'aldéhyde hexyl-cinnamique et de l'acétate de menthyle à un rapport de 1:1 en poids.

15. Article absorbant selon l'une quelconque des revendications précédentes, ledit article absorbant étant une serviette hygiénique, un protège-slip, ou un produit pour l'incontinence légère des adultes, et comprenant en outre un moyen adhésif sur ledit côté faisant face au vêtement de ladite feuille de fond, et une couche de libération qui y est appliquée.
